# EUROPEAN PATENT SPECIFICATION

(11) **EP 1 801 582 B1**
(45) Date of publication and mention of the grant of the patent: **17.03.2010**
(21) Application number: 05780351.2
(22) Date of filing: 19.08.2005
(51) Int. Cl.: G01N 33/50

(54) **METHOD OF PREDICTING THE METABOLISM OF DRUG IN HUMAN LIVER AND LIVER FUNCTION**
VERFAHREN ZUR ABSCHÄTZUNG DES METABOLISMUS EINES ARZNEISTOFFS IN DER MENSCHLICHEN LEBER SOWIE DER LEBERFUNKTION
PROCEDE DE PRESOMPTION DU METABOLISME DE MEDICAMENT DANS LE FOIE HUMAIN ET FONCTION HEPATIQUE

(30) Priority: 20.08.2004 JP 2004240827
(43) Date of publication of application: 27.06.2007
(73) Proprietor: Sekisui Medical Co., Ltd., Tokyo 103-0027 (JP); PhoenixBio Co., Ltd., Higashihiroshima-shi Hiroshima 739-0046 (JP)
(72) Inventor: NINOMIYA, Shin-ichi, Naka-gun, Ibaraki 3191182 (JP); OHZONE, Yoshihiro, Naka-gun, Ibaraki 3191182 (JP); ADACHI, Yasuhisa, Naka-gun, Ibaraki 3191182 (JP); HORIE, Toru, PhoenixBio Co., Ltd., Higashihiroshima-shi, Hiroshima 7390046 (JP); SOENO, Yoshinori, PhoenixBio Co., Ltd., Higashihiroshima-shi, Hiroshima 7390046 (JP); INOUE, Tae, PhoenixBio Co., Ltd., Higashihiroshima-shi, Hiroshima 7390046 (JP)
(74) Representative: Hartz, Nikolai
(86) International application number: PCT/JP2005/015148
(87) International publication number: WO 2006/019162

(56) References cited:
- EP-A- 1 552 740
- WO-A-01/32009
- WO-A-01/67854
- WO-A-03/000848
- WO-A1-00/03001
- WO-A1-94/02601
- WO-A1-03/080821
- WO-A2-00/43498
- JP-A- 2002 045 087
- TATENO C ET AL: "Near completely humanized liver in mice shows human-type metabolic responses to drugs" AMERICAN JOURNAL OF PATHOLOGY, PHILADELPHIA, PA, US, vol. 165, no. 3, September 2004 (2004-09), pages 901-912, XP002996759 ISSN: 0002-9440

## Description

### Technical Field

The present invention relates to a method for analyzing a human-specific metabolite of a drug in human liver and to a method for determining an animal exhibiting a human-type metabolic profile for a test substance.

### Background Art

Many substances which have been ingested by the human body are initially metabolized in the liver. Particularly in the development of pharmaceuticals, the metabolic mechanism of a drug in the liver and the effect of the drug on liver function are essential data from the aspect of safety. In addition to the case of pharmaceuticals, in order to evaluate the effects on the human body of a variety of chemical substances present in the environment, the effects of the chemical substances on liver function must be evaluated.

In the development of pharmaceuticals or other substances, the metabolism of a test substance in the human liver and its effect on human liver function cannot be determined through direct administration thereof to the human subjects. Thus, conventionally, mammals including rats, mice, rabbits, dogs, and chimpanzees have been employed to test such substances. Since drug metabolism in the liver is known to vary considerably depending on the animal species, it is difficult to predict the metabolism in humans from the test results obtained from non-human animals.

In such circumstances, recently, *in vitro* assay systems have been developed and used, such as a system employing microsomes which express human drug metabolizing enzymes and a system employing human hepatocytes. However, these in vitro assay systems have drawbacks, for example, in that the results vary depending on addition of a co-enzyme, the type of the assay system, etc. Therefore, at present, metabolism through the human liver cannot be precisely predicted.

As an animal used for *in vivo* studies to measure the metabolism of a test substance in the liver or the like, there have been reported chimeric animals which carry a population of human-origin hepatocytes and in which the hepatocyte population substantially functions as the liver of the chimeric animals (Patent Documents 1 and 2).
Patent Document 1: Japanese Laid-Open Patent Publication No. 2002-45087
Patent Document 2: WO 03/080821 A1
WO 03/080821 A1 describes a method of proliferating human hepatocytes and a method of obtaining hepatocytes.
EP 1 552 740 A1 describes a chimeric murine model comprising human hepatocytes and a method for the *in vivo* study of metabolism of administered compounds.
WO 01/32009 A1 relates to the preparation of non-human animals having chimeric livers having human hepatocytes.

### Disclosure of the Invention

### Problems to be Solved by the Invention

However, the aforementioned Patent Document 1 fails to provide examples in which a test substance is administered to chimeric animals. Therefore, drug metabolism of the chimeric animals after a test substance is actually administered to them remains unclear. In addition, possible adverse effects of the test substance cannot be predicted. Actual trials have revealed that administration of a test substance to the chimeric animals causes blood coagulation and the like anomalously. Since these chimeric animals have impaired their liver functions, the value of their liver-function markers are abnormal. Thus, it has never been determined whether or not the variation in liver-function markers after a test drug has been administered is attributed to the effect of the test drug on the transplanted human hepatocytes. In addition, it has not been able to determine whether the measured values after administration of a test substance are completely attributable to transplanted human hepatocytes or it also attribute to inherent hepatocytes of the animals.

An object of the present invention is to provide an *in vivo* method for precisely predicting the metabolism of a drug in human liver, the effect of the drug on liver function, and so on.

### Means for Solving the Problems

The present inventors have conducted extensive studies in order to attain the object, and we found that when a test substance is administered to a chimeric animal transplanted with human hepatocytes, if human complement reaction can be suppressed in advance, the chimeric animal can be maintained in a favorable state. We also found that metabolites of a test substance produced in human liver and their effect on the liver functions can be precisely predicted by administrating the test substance to both the chimeric animal transplanted with the human hepatocytes and a non-chimeric animal of the same species, and by analyzing and comparing metabolites and function of the liver in these animals. The present invention has been established on the basis of these findings.

Accordingly, the present invention provides a method for analyzing a human-specific metabolite of a test substance,
wherein the method comprises:
(i) administering a test substance to a chimeric animal and a non-chimeric animal of the same species;
   wherein said chimeric animal is a mouse derived from an uPA-Tg(+/+)/SCID(+/+) mouse and intracorporeally carries a population of human-origin hepatocytes having proliferation potential and in which the hepatocyte population substantially functions as the liver of the chimeric animal;
(ii) protecting the chimeric animal from the attack by a human complement produced by the hepatocytes by administering a complement suppressor or an anticoagulant to the chimeric animal;
(iii) analyzing metabolites from the chimeric animal and the non-chimeric animal;
(iv) analyzing metabolites of the test substance from an animal of a species other than the chimeric animal; and
(v) comparing the analytical results.
   The invention further provides a method for determining an animal exhibiting a human-type metabolic profile for a test substance, wherein the method comprises:

(i) administering a test substance to a chimeric animal and a non-chimeric animal of the same species;
   wherein said chimeric animal is a mouse derived from an uPA-Tg(+/+)/SCID(+/+) mouse and intracorporeally carries a population of human-origin hepatocytes having proliferation potential and in which the hepatocyte population substantially functions as the liver of the chimeric animal;
(ii) protecting the chimeric animal from the attack by a human complement produced by the hepatocytes by administering a complement suppressor or an anticoagulant to the chimeric animal;
(iii) analyzing metabolites from the chimeric animal and the non-chimeric animal;
(iv) analyzing metabolites of the test substance from an animal of a species other than the chimeric animal; and
(v) comparing the analytical results.

Also described is a method for predicting a metabolite of a test substance produced in human liver, wherein the method comprises:
administering a test substance to a nonhuman chimeric animal and a non-chimeric animal of the same species, wherein the chimeric animal intracorporeally carries a population of human-origin hepatocytes having proliferation potential and in which the hepatocyte population substantially functions as the liver of the chimeric animal, and wherein these animals are protected from the attack by a human complement produced by the hepatocytes; and
analyzing and comparing metabolites from the chimeric animal and the non-chimeric animal.

Also described is a method for determining the type of excretion for a test substance in human, wherein the method comprises:
administering a test substance to a nonhuman chimeric animal and a non-chimeric animal of the same species, wherein the chimeric animal intracorporeally carries a population of human-origin hepatocytes having proliferation potential and in which the hepatocyte population substantially functions as the liver of the chimeric animal, and wherein these animals are protected from the attack by a human complement produced by the hepatocytes; and
analyzing and comparing metabolites from the chimeric animal and the non-chimeric animal.

Also described is a method for predicting an effect of a test substance on liver functions in human, wherein the method comprises:
administering a test substance to a nonhuman chimeric animal and a non-chimeric animal of the same species, wherein the chimeric animal intracorporeally carries a population of human-origin hepatocytes having proliferation potential and in which the hepatocyte population substantially functions as the liver of the chimeric animal, and wherein these animals are protected from the attack by a human complement produced by the hepatocytes; and
examining and comparing liver functions of the chimeric animal and the non-chimeric animal.

Also described is a method for analyzing a human-specific metabolite of a test substance,
wherein the method comprises:
administering a test substance to a nonhuman chimeric animal and a non-chimeric animal of the same species, wherein the chimeric animal intracorporeally carries a population of human-origin hepatocytes having proliferation potential and in which the hepatocyte population substantially functions as the liver of the chimeric animal, and wherein these animals are protected from the attack by a human complement produced by the hepatocytes;
analyzing metabolites from the chimeric animal and the non-chimeric animal;
analyzing metabolites of the test substance from an animal of a species other than the chimeric animal; and
comparing the analytical results.

Further described is a method for predicting an animal exhibiting a human-type metabolic profile for a test substance, wherein the method comprises:
administering a test substance to a nonhuman chimeric animal and a non-chimeric animal of the same species, wherein the chimeric animal intracorporeally carries a population of human-origin hepatocytes having proliferation potential and in which the hepatocyte population substantially functions as the liver of the chimeric animal, and wherein the animals are protected from the attack by a human complement produced by the hepatocytes;
analyzing metabolites from the chimeric animal and the non-chimeric animal;
analyzing metabolites of the test substance from an animal of a species other than the chimeric animal; and
comparing the analytical results.

### Effects of the Invention

According to the present invention, metabolism in the liver, the type of excretion, an effect on liver functions and the like of a drug in human can be precisely predicted *in vivo* analysis. Thus, according to the present invention, the effect of a drug on the human body can be predicted before their administration to human, and screening of candidates and prediction of side effects or potency of the drug in human can be facilitated during the course of the development of pharmaceuticals. Therefore, the invention allows reducing development cost by narrowing down the candidates in an early stage of drug development, and preventing side effects in human. Thus, the present invention is remarkably useful in the development of pharmaceuticals.

### Brief Description of the Drawings

[Fig. 1] A graph showing time-elapse change in radioactivity levels of blood plasma samples.
[Fig. 2] A graph showing excretion in bile.
[Fig. 3] Graphs showing percent excretion of radioactivity in urine and feces.
[Fig. 4] Graphs showing predominant peaks observed in blood plasma samples two hours after administration.
[Fig. 5] Chromatograms of radioactivity in bile.
[Fig. 6] Chromatograms of radioactivity in liver and kidney, recorded two hours after administration.
[Fig. 7] Chromatograms of radioactivity in feces.
[Fig. 8] Chromatograms of radioactivity in urine.

### Best Modes for Carrying Out the Invention

The nonhuman chimeric animal employed in the present invention is a chimeric animal which intracorporeally carries a population of human-origin hepatocytes having proliferation potential and in which the hepatocyte population substantially functions as the liver of the chimeric animal. The chimeric animal is preferably an immunodeficient animal whose original hepatocytes have been impaired and which exhibits no rejection against human hepatocytes (i.e., an immunodeficient animal with hepatic disorder), wherein the immunodeficient animal has been transplanted with human hepatocytes, and engraftment of the human hepatocytes has been confirmed.

The animal with hepatic disorder employed in the present invention may be a transgenic mouse disclosed in Science, 263, 1149(1994), to which an albumin promoter and a fusion gene of an enhancer region and u-PA (urokinase-type plasminogen activator) have been introduced. The immunodeficient animal may be an SCID mouse (severe combined immunodeficiency disease mouse), which is genetically immunodeficient. In a simple manner, progeny mice obtained through mating the above hepatic impaired mouse and the immunodeficient mouse may be employed as an immunodeficient animal with hepatic disorder. The species of these animals is mouse. Examples of preferred immunodeficient mice with hepatic disorder include uPA-Tg (+/+)/SCID mice.

The human hepatocytes for transplanting into the immunodeficient animal with hepatic disorder are preferably frozen hepatocytes, fresh hepatocytes, and small liver parenchymal cells with high proliferation potential. Interracial difference in the metabolism can be evaluated by transplanting the hepatocytes from Japanese, Korean, Chinese, Caucasians, Negroids, or people of other races.

Preferably, human hepatocytes are transplanted to the spleen of the immunodeficient animal with hepatic disorder. Once human hepatocytes have been transplanted into the spleen, the cells are readily engrafted successfully into the liver.

The chimeric animal employed in the present invention is a mouse derived from an uPA-Tg(+/+)/SCID(+/+) mice disclosed in Japanese Laid-Open Patent Publication No. 2002-45087, wherein this mouse have been transplanted with human hepatocytes to the spleen and engraftment of the human hepatocytes has been confirmed.

The non-chimeric animal of the same species as the chimeric animal employed in the invention is preferably an immunodeficient animal with hepatic disorder, more preferably an immunodeficient mouse with hepatic disorder, particularly preferably an uPA-Tg(-/-)/SCID(+/+) mouse, an uPA-Tg(+/)/SCID(+/+) mouse, or an uPA-Tg(+/+)/SCID(+/+) mouse.

In the present invention, a test substance is administered to a chimeric animal and to a non-chimeric animal of the same species, while these animals are protected from the attack by a human complement produced by the hepatocytes. This is because when the animals are not protected from the attack by a human complement, administration of the test substance leads to onset of disseminated coagulation syndrome or the like. In order to maintain a state in which the animals are protected from the attack by a human complement, a complement suppressor (e.g., nafamostat mesylate) or an anticoagulant for preventing blood coagulation (e.g., heparin) is preferably administered in advance to the animals. No particular limitation is imposed on the amount of such agents, so long as anticoagulation effect can be attained. These agents are administered before administration of a test substance.

In the present invention, a test substance is administered to both a chimeric animal and a non-chimeric animal of the same species. The test substance may be administered through the means most common to the substance. However, in consideration of difference in absorption capacity among species, the test substance is preferably administered intravenously, orally, or the like.

After administration of the test substance, metabolites of the chimeric animal and the non-chimeric animal are analyzed and the analytical results are compared, whereby the metabolites of the test substance produced in human liver can be precisely predicted. The term "analyzing metabolite(s)" refers to obtaining information including the type, amount and composition of the metabolites, and time-elapse or quantitative change thereof. Analysis of metabolites from plasma samples, tissue samples, bile samples, urine samples, and feces samples are preferred. A convenient means for analyzing the metabolites is using a substance containing a radioisotope as a test substance, to measure radioactivity of the test substance. The metabolites include, for example a variety of degraded products and conjugates.

According to the present invention, using both chimeric animals and non-chimeric animals to compare their analytical results allows to find the action of test substances on human hepatocytes in the chimeric animal precisely, even if the percent expression of the function of human hepatocytes in the chimeric animal is several tens. In addition, hepatic disorder of the chimeric animal *per se* can be distinguished from hepatic disorder caused by the test substance. Furthermore, through determination of changes in mRNA, proteins, and other substance in the liver by various methods, the mechanism of disorder in liver function or that of hepatotoxicity can be elucidated.

After administration of the test substance, metabolites of the chimeric animal and the non-chimeric animal are analyzed, and the analytical results are compared, whereby the type of excretion of the test substance in human can be determined. Specifically, for example, metabolites in urine samples, feces samples, and bile samples of both animals can be measured and compared to determine the type of excretion of a test substance in human. The type of excretion greatly varies among animal species. Therefore, in development of pharmaceuticals, determination of the type of excretion of the test substance in human is extremely important. Thus, the present invention, which enables to evaluate the type of excretion or metabolites of a test substance in human without administering the substance to human subjects, is remarkably useful.

After administration of the test substance, functions of the liver of the chimeric animal and those of the non-chimeric animal are examined and compared, whereby the effect of a test substance on liver function in human can be precisely predicted; including a prophylactic or therapeutic effect on hepatopathy and a toxicity to hepatocytes. Since the chimeric animals have impaired liver functions, and thus the value of liver-function markers (e.g., GOT and GPT) are abnormal before administration of a test substance, a control animal must be employed with the chimeric animal, so as to elucidate the effect of the test substance on liver functions in human. The control animal is preferably a non-chimeric animal of the same species; e.g., an immunodeficient animal with hepatic disorder, an immunodeficient animal, or an animal having no disorder.

Examples of test items for liver function include biochemical scores such as GOT and GPT. Alternatively, changes in mRNA, expressed proteins, and other substance in the liver may be determined through a known method.

As described above, after administration of a test substance, metabolites of a chimeric animal and those of a non-chimeric animal are analyzed and compared, whereby the excretion type or metabolites of the test substance in human are evaluated. Subsequently, metabolites of the test substance of an animal species other than the chimeric animal are analyzed, and then these analytical results are compared, whereby an animal exhibiting a human-type metabolic profile for the test substance can be predicted. In other words, according to the invention, it can be readily determined in a variety of animals whether or not the metabolic profile for the test substance is a human-type. These results can be used in determining adaptability of the animal for employment in the studies on the effect of the test substance and metabolites thereof.

### Examples

The present invention will be described hereinafter in detail by way of examples, which should not be construed as limiting to the invention.

### Example 1

### A. Materials

### (1) Test substance

Ketoprofen was employed as a test substance, which is known to be mainly excreted in urine as a glucuronate conjugate in human.

³H-ketoprofen (ART391, Lot 040219) was purchased from ARC. The compound was generally labeled and had a specific radioactivity of 1,110 GBq/mmol.

### (2) Animals employed

Chimeric mice from PhoenixBio Co., Ltd. were employed. Specifically, human hepatocytes were transplanted to the spleen of the transgenic mice with liver failure (uPA-Tg (+/+)/SCID mice (female)), and the mice in which engraftment of human hepatocytes was recognized were employed (body weight: 10.2 to 18.0 g). Mice employed had percent replacement of the human hepatocytes therein of 60% to 80%, and caged for 45 days to 87 days after transplantation. uPA (-/-)/SCID mice of 6 to 10 weeks aged (body weight: 15.1 to 22.2 g) were employed as control mice.
The thus-employed mice were freely fed water (containing 0.012% hypochlorous acid) and a sterilized solid diet containing vitamin C (CRF-1, product of Oriental Yeast Co., Ltd.) and housed at room temperature (23 ± 3°C) and a humidity of 55 ± 20%. The chimeric mice were injected intraperitoneally with nafamostat mesylate twice per day (0.3 mg/0.2 mL/body).

### (3) Dose and method of administration

A predetermined amount of a ³H-ketoprofen ethanol solution was dried to solid under nitrogen flow. Subsequently, a 0.125-mol/L solution of ketoprofen in sodium hydroxide was added to dissolve the solid. The pH of the solution was adjusted to 7.0 with a 0.1% aqueous citric acid solution, and the concentration was adjusted to 1 mg/mL with distilled water for injection. The liquid was bolus-administered at 5 mL/kg to the caudal vein of the mice (5 mg/5 mL/kg).

### (4) Collection of samples

Urine and feces were collected from chimeric mice and control mice which had been housed in metabolism cages, over 5 days after administration. Livers and kidneys were taken from other mice two hours after administration, following sacrificing through exsanguination. Blood plasma was collected by sampling blood through the orbital venousplexus and centrifuging it. Bile was collected from the chimeric mice and the control mice through bile duct cannulation until 72 hours after administration.

### (5) Measurement of radioactivity

Radioactivity of each sample was determined by use of a liquid scintillator (Hionic-flow, product of Perkin-Elmer) with Tri-Carb 2500 (Perkin-Elmer) as a liquid scintillation counter. The measurement was performed for two minutes.
Counting efficiency was calibrated through the external standard radiation source method.

(6) Determination of metabolite concentrations in blood plasma, tissues, bile, urine, and feces
In analysis of blood plasma, bile, and urine, the sample (10 µL) was collected in a 20-mL glass vial, added Hionic-flow (10 mL), and the mixture was analyzed.
In analysis of tissue samples (liver and kidney), an amount 3-fold (by weight) of acetate buffer (0.1M, pH3.0) was added to each sample, and the mixture was homogenized with a polytron-homogenizer. An aliquot (50 µL) of the homogenate was solubilized with 2 mL of Soluene 350 (Perkin-Elmer), added Hionic-flow (10 mL), and the mixture was analyzed.
In analysis of feces samples, the total volume of each sample was adjusted to 15 or 30 mL. The mixture was homogenized with a polytron-homogenizer. An aliquot (0.5 mL) of the homogenate was solubilized with 2 mL of Soluene 350 (Perkin-Elmer), added Hionic-flow (10 mL), and the mixture was analyzed.

### (7) Analysis of metabolites in blood plasma, tissues, and urine

### (i) Analysis of blood plasma, tissue, and feces

A plasma sample (50 µL) or a tissue homogenate (100 µL) was added to an Eppendorf tube, and acetonitrile was added in an amount 4-fold of the sample, followed by vigorously stirring. The mixture was centrifuged (22,000×g, 4°C, 10 min), and the supernatant was collected and dried to solid under nitrogen flow. The solid was re-dissolved with a 200 µL of acetate buffer (0.1M, pH3.0), and the solution was centrifuged again (22,000xg, 4°C, 10 min). The collected supernatant was applied to an HPLC column.
An aliquot (500 µL) of the feces homogenate was collected in a glass tube, and acetonitrile was added in an amount 9-fold of the sample, followed by vigorously stirring. The mixture was centrifuged (1,800xg, 4°C, 10 min), and the supernatant was collected. Distilled water (500 µL) was added to the residue, and the mixture was stirred. The above extraction with acetonitrile was repeated. The obtained supernatants were combined and dried to solid under nitrogen flow. The solid was re-dissolved with a 200 µL of acetate buffer (0.1M, pH3.0), and the solution was centrifuged (22,000×g, 4°C, 10 min). The collected supernatant was applied to an HPLC column.

### (ii) Analysis of bile and urine

An aliquot (25 µL) of the bile sample was diluted with an acetate buffer (0.1M, pH3.0) (75 µL), and ultrafiltered (22,000xg, 4°C, 10 min) with an Ultrafree (0.45 µm, product of Nihon Millipore Ltd.). The filtrate was applied to an HPLC column.
The urine sample was collected into a urine-collection tube attached to each metabolism cage, to which an acetate buffer (0.1M, pH3.0) (200 µL or 500 µL) had been added in advance. The sample (100 µL) was ultrafiltered (22,000xg, 4°C, 10 min) with an Ultrafree (0.45 µm, product of Nihon Millipore Ltd.), and the filtrate was applied to an HPLC column.

### (iii) Enzyme treatment

Bile samples and urine samples were treated with β-glucuronidase-arylsulfatase (Helix pomatia, product of Roche Diagnoistics K.K.). Specifically, β-glucuronidase-arylsulfatase (3%) was added to each of the bile and urine samples, and the sample was incubated at 37°C for 16 hours.
The incubated sample was centrifuged (22,000xg, 4°C, 10 min), and the supernatant was applied to an HPLC column.

### (iv) Condition for HPLC analysis

HPLC system: Shimadzu LC-10 Avp series
Column: Inertsil ODS-3, 5 µm, 4.6 mm × 150 mm (product of GL science Inc.)
Mobile phase: Liquid A) 0.1% aqueous acetic acid solution Liquid B) 0.1% acetonitrile
Gradient: Time (min) 0→15→25→26→35 B conc. (%) 25→80→80→25→25
Column temperature: 40°C
Detection: UV detector (254 nm) and continuous radioactivity detector

### B. Test results

### (1) Determination of metabolite concentrations in blood plasma, tissues, bile, urine, and feces

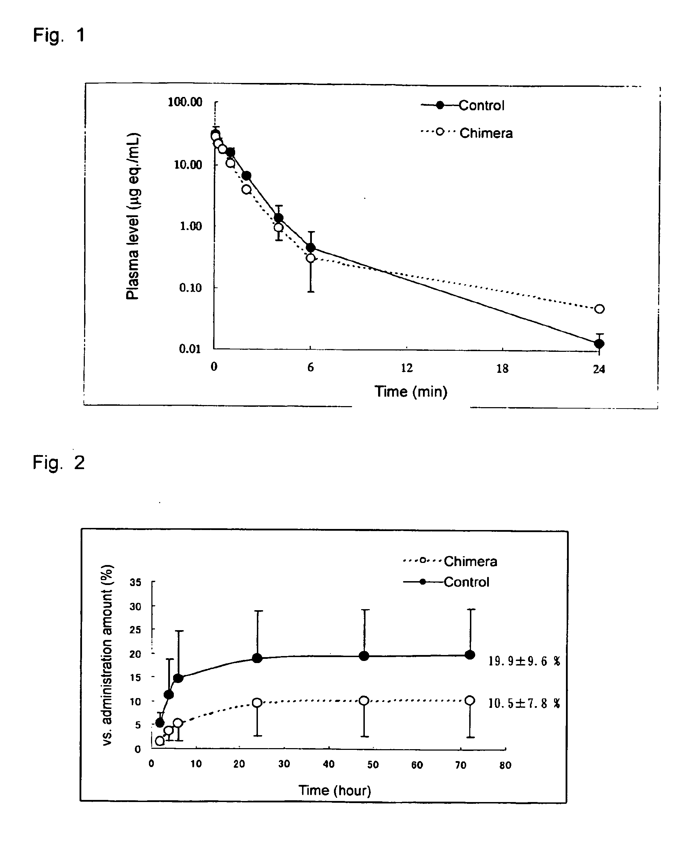

Fig. 1 shows time-elapse change in radioactivity levels of blood plasma, and Table 1 shows kinetic parameters obtained through non-compartment analysis. In the control mice (n=2) and the chimeric mice (n=3), AUCs (on the basis of total radioactivity) were 45.8 µg eq.·h/mL and 35.7 µg eq.·h/mL, respectively, and t1/2 (0-4 h) were 0.92 h and 0.78 h, respectively. AUC level were almost equivalent between the two mice groups.

**[Table 1]**

| | Tₘₐₓ (min) | Cₘₐₓ (µg eq./mL) | t1/2 (0-4 hr) | t1/2 (6-24 hr) | AUC (µg eq.hr/mL) | Vd (mL/kg) | CL total (mL/h/kg) |
|---|---|---|---|---|---|---|---|
| Control (ave.) | | 30.8 | 0.92 | 3.72 | 45.8 | 37.8 | 22.0 |
| Chimera (ave.) | | 27.5 | 0.78 | 6.81 | 35.7 | 56.7 | 29.8 |

Fig. 2 shows excretion in bile. Percent excretions (relative to administration) to bile in the control mice (n=3) and the chimeric mice (n=3) were 19.9 ± 9.6% and 10.5 ± 7.8%, respectively. No significant difference was found between the two mice groups, although the control mice exhibited a slightly higher value.

Regarding radioactivity levels of tissue samples determined at two hours after administration, radioactivity levels of the control mice (n=3) and the chimeric mice (n=3) were 2.58 ± 0.33 µg eq./mL and 2.14 ± 0.98 µg eq./mL in liver, and 4.70 ± 0.61 µg eq./mL and 5.03 ± 1.25 µg eq./mL in kidney, respectively (see Table 2). Radioactivity levels of blood plasma samples in the control mice and the chimeric mice determined at the same time were 6.82 ± 1.10 µg eq./mL and 6.96 ± 2.65 µg eq./mL, respectively (Table 2). In terms of the radioactivity levels of the tissue samples and blood plasma samples, no significant difference was found between the two mice groups. Ratio (tissue to plasma) in radioactivity level (Kp) were calculated and revealed that the ratio Kp was significantly lowered in liver of the chimeric mice, as compared with the control mice (Table 2).

**[Table 2]**

| | Concentration (µg eq./mL) | | | |
|---|---|---|---|---|
| | Control mice | | Chimeric mice | |
| | ave. | SD | ave. | SD |
| Liver | 2.58 | 0.33 | 2.14 | 0.98 |
| Kidney | 4.70 | 0.61 | 5.03 | 1.25 |
| Plasma | 6.82 | 1.10 | 6.96 | 2.65 |

| | | Kp (g tissue/mL) | | |
|---|---|---|---|---|
| | Control mice | | Chimeric mice | |
| | ave. | SD | ave. | SD |
| Liver | 0.380 | 0.021 | 0.301* | 0.033 |
| Kidney | 0.700 | 0.147 | 0.777 | 0.292 |

| | | | | |
|---|---|---|---|---|
| *; p<0.05 | | | | |

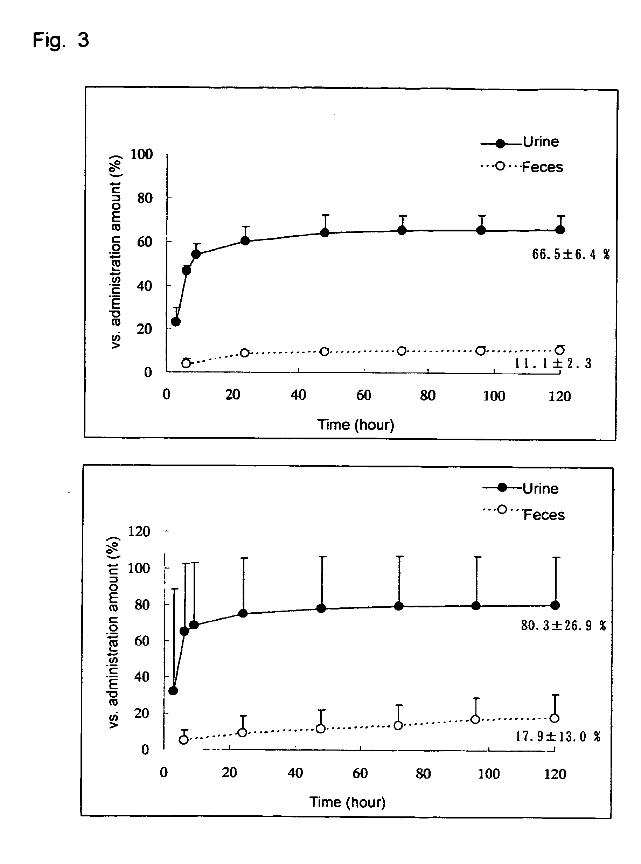

Fig. 3 shows percent excretions of radioactivity to urine and feces. The percent excretion (relative to administration) to urine until 120 hours after the administration in the control mice (n=3) and the chimeric mice (n=3) were found to be 66.5 ± 6.4% and 80.3 ± 26.9%, respectively. The percent excretion (relative to administration) to feces until 120 hours after the administration in the control mice (n=3) and the chimeric mice (n=3) were found to be 17.9 ± 13.0% and 11.1 ± 2.3%, respectively. In the chimeric mice, radioactivity was more preferentially excreted in urine.

### (2) Analysis of metabolites in blood plasma, tissues, and urine

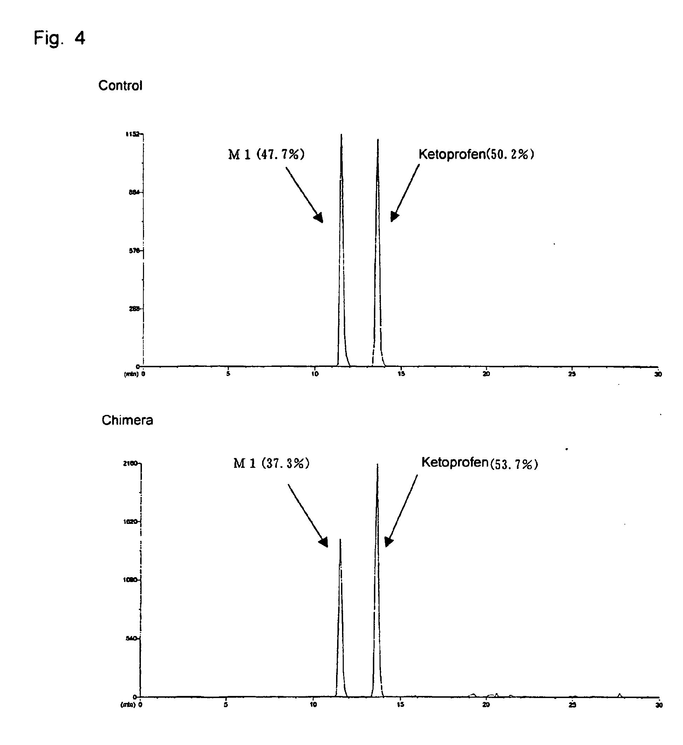

As shown in Fig. 4, both of the control mice and the chimeric mice predominantly exhibited a peak of an unchanged form and a hydroxyl metabolite (speculated, hereinafter referred to as M1) in their blood plasma samples collected two hours after the administration. Formation of M1 was more predominant in the control mice than in the chimeric mice.

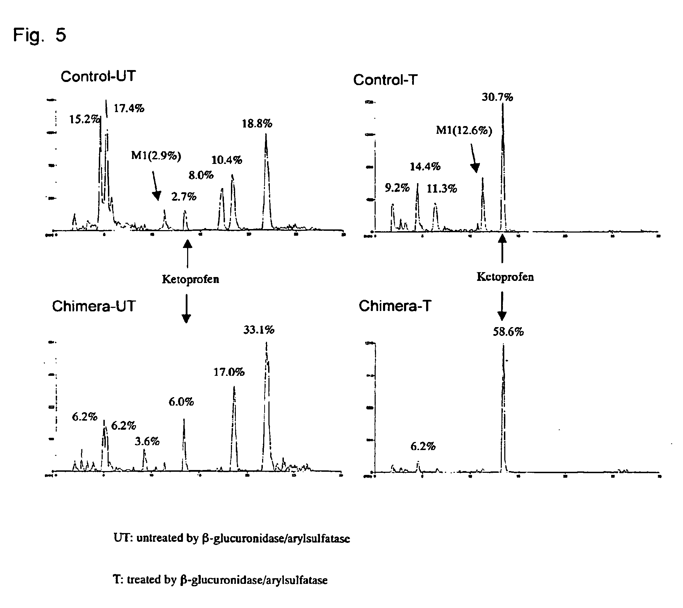

Fig. 5 shows chromatograms of radioactivity in bile samples. In bile samples, the unchanged form was not virtually observed, and peaks attributable to glucuronide conjugates were observed. Through enzyme treatment, the control mice exhibited some peaks attributable to a hydroxyl metabolite, whereas the chimeric mice exhibited a predominant peak attributed to the unchanged form.

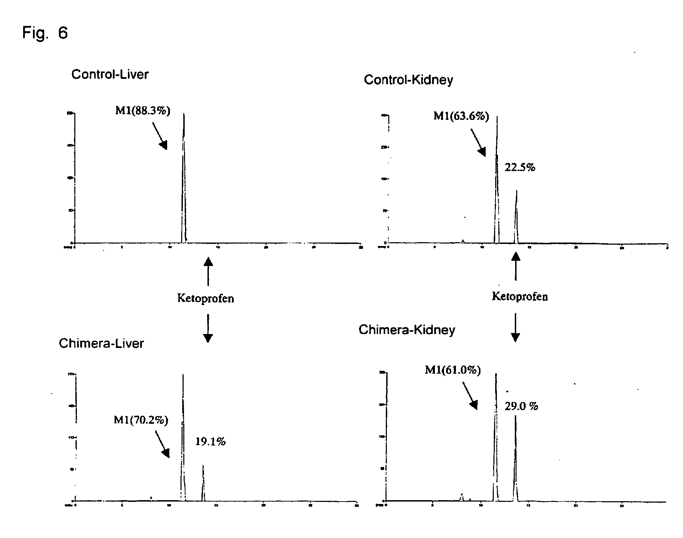

Regarding tissue samples collected two hours after the administration, a predominant peak attributed to M1 was observed and no peak attributable to the unchanged form was observed in liver samples of the control mice (see Fig. 6). In contrast, liver samples of the chimeric mice exhibited peaks attributed to the unchanged form and M1, respectively. As shown in Fig. 6, peaks attributed to the unchanged form and M1 were observed in kidney from both of the control mice and the chimeric mice. In both of the liver and the kidney, formation of M1 was more predominant in the control mice as compared with the chimeric mice.

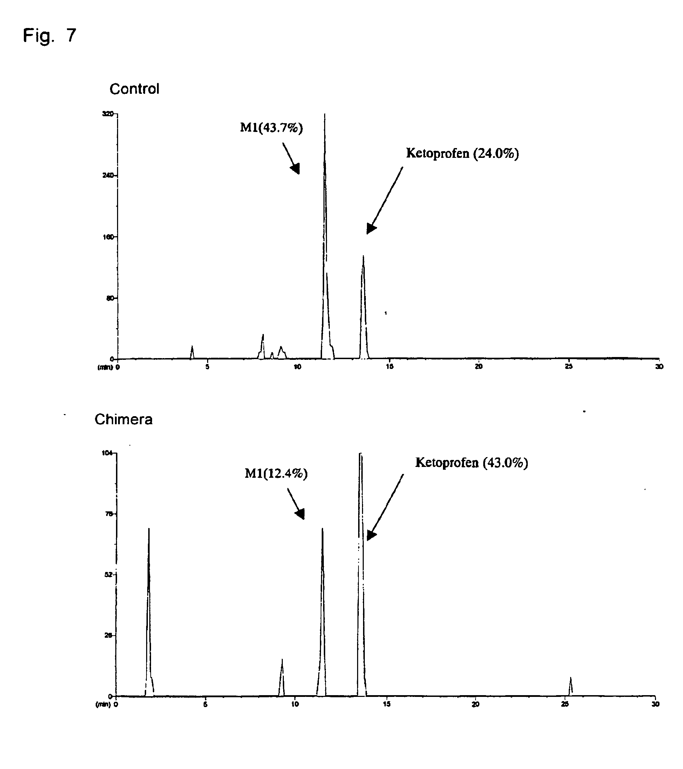

Since feces samples exhibited low radioactivity level, the chromatography of radioactivity was heavily noisy. Nevertheless, peaks attributed to the unchanged form and M1 were observed as predominant peaks, both in the control mice and the chimeric mice (see Fig. 7).

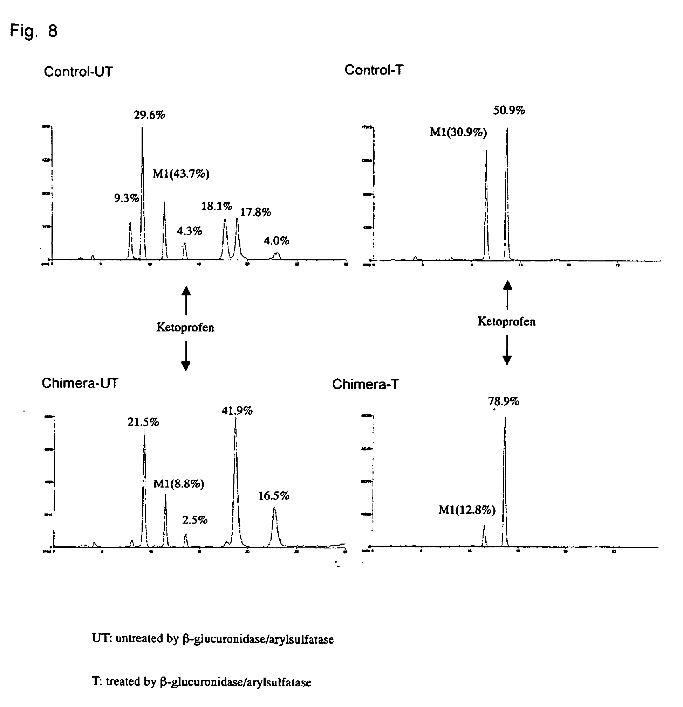

Fig. 8 shows typical chromatograms of the urine samples. In the urine samples of the control mice and the chimeric mice, a number of peaks attributable to the unchanged form, a hydroxyl metabolite, and conjugate thereof were observed. Particularly, in the case of the control mice, the peak eluted at 17.8 min was considered to be a peak intrinsic to mice. Peaks in the chromatogram of radioactivity were found to be changed to those attributed to the unchanged form and M1, when the urine samples were treated with β-glucuronidase-arylsulphatase. Formation of a hydroxyl metabolite was more predominant in the control mice as compared with the chimeric mice.

When the samples were treated with acetonitrile in a pre-analysis stage, percent recovery of radioactivity in blood plasma samples, tissue (liver and kidney) samples and feces samples were all as high as 88.5 to 95.7%. Since the percent recovery was at a satisfactory level, the acetonitrile treatment was considered to not cause any problem in the determination of the metabolic profile.

(1) As described above, no clear difference was found, in terms of time-elapse change in metabolite concentration in blood plasma and tissue samples, between the control mice and the chimeric mice. However, percent excretion in bile was higher in the control mice than in the chimeric mice. Since the excretion balance factor (urine/feces) was varied in a wide range, no significant difference was observed between the two mice groups. However, percent excretion in urine was higher in the chimeric mice than in the control mice. This feature was attributable to the fact that substances including metabolites are excreted in urine more preferentially in human than in rodents, indicating that chimeric mice exhibit excretion function to bile more similar to that of human.

(2) Through comparison of the metabolic profiles of urine samples and bile samples, a conjugate presumably formed predominantly from the unchanged compound was observed in the samples from the chimeric mice, whereas the presence of a hydroxyl metabolite and a conjugate thereof was confirmed in the control mice, indicating that the chimeric mice exhibited a metabolic profile more similar to that of human.
Therefore, these results indicate that the excretion type of the test substance in human (e.g., whether the type is urine excretion or bile excretion) can be predicted by administering a test substance to a nonhuman chimeric animal and a non-chimeric animal of the same species, wherein the chimeric animal intracorporeally carries a population of human-origin hepatocytes and in which the hepatocyte population substantially functions as the liver of the chimeric animal, and wherein these animals are protected from the attack by a human complement produced by the hepatocytes, and analyzing and comparing metabolites from the chimeric animal and the non-chimeric animal. In addition, when the excretion type is compared with that of other animal species, the presence of a human-specific metabolite in the chimeric animal can be confirmed, or it can be determined whether the metabolic profile of the other animal is identical to that of human or not.

(3) In the case where a 2-phase metabolic enzyme (in particular glucuronide conjugation) predominantly participates in metabolism, extrapolation of data obtained through in vitro tests to *in vivo* situation has been reported to be difficult. When human hepatocytes were employed, formation of a hydroxyl metabolite has been observed, but no formation of a conjugated metabolite. However, according to the present invention, conjugated metabolites have been able to be confirmed, which have never been confirmed in *in vitro* tests employing human hepatocytes, indicating that the present invention is of remarkably great value.

## Claims

1. A method for analyzing a human-specific metabolite of a test substance,
wherein the method comprises:
(i) administering a test substance to a chimeric mouse and a non-chimeric mouse of the same species;
wherein the chimeric mouse is derived from a uPA-Tg(+/+)/SCID(+/+) mouse and is transplanted with human-origin hepatocytes having proliferation potential and in which the hepatocyte population substantially functions as the liver of the chimeric mouse;
(ii) protecting the chimeric mouse from the attack by a human complement produced by the hepatocytes by administering a complement suppressor or an anticoagulant to the chimeric mouse;
(iii) analyzing metabolites from the chimeric mouse and the non-chimeric mouse;
(iv) analyzing metabolites of the test substance from an animal of a species other than the chimeric mouse; and
(v) comparing the analytical results.

2. A method for determining an animal exhibiting a human-type metabolic profile for a test substance, wherein the method comprises:
(i) administering a test substance to a chimeric mouse and a non-chimeric mouse of the same species;
wherein the chimeric mouse is derived from a uPA-Tg(+/+)/SCID(+/+) mouse and is transplanted with human-origin hepatocytes having proliferation potential and in which the hepatocyte population substantially functions as the liver of the chimeric mouse;
(ii) protecting the chimeric mouse from the attack by a human complement produced by the hepatocytes by administering a complement suppressor or an anticoagulant to the chimeric mouse;
(iii) analyzing metabolites from the chimeric mouse and the non-chimeric mouse;
(iv) analyzing metabolites of the test substance from an animal of a species other than the chimeric mouse; and
(v) comparing the analytical results.

## Patentansprüche

1. Verfahren zur Analyse eines Menschen-spezifischen Metaboliten einer Testsubstanz, wobei das Verfahren umfasst:
(i) Verabreichen einer Testsubstanz an eine chimäre Maus und an eine nichtchimäre Maus derselben Art; wobei die chimäre Maus von einer uPA-Tg(+/+)/SCID(+/+)-Maus abstammt und mit Hepatozyten menschlichen Ursprungs transplantiert ist, die Vermehrungspotential haben, und in der die Hepatozytenpopulation im wesentlichen als die Leber der chimären Maus fungiert;
(ii) Schützen der chimären Maus vor dem Angriff durch ein humanes Komplement, das von den Hepatozyten produziert wird, durch Verabreichen eines Komplementsuppressors oder eines Antikoagulans an die chimäre Maus;
(iii) Analysieren von Metaboliten der chimären Maus und der nichtchimären Maus;
(iv) Analysieren von Metaboliten der Testsubstanz eines Tieres einer anderen Art als der der chimären Maus; und
(v) Vergleichen der Analyseeergebnisse.

2. Verfahren zur Bestimmung eines Tieres, das ein menschenartiges Metabolismusprofil bezüglich einer Testsubstanz aufweist, wobei das Verfahren umfasst:
(i) Verabreichen einer Testsubstanz an eine chimäre Maus und eine nichtchimäre Maus derselben Art; wobei die chimäre Maus von einer uPA-Tg(+/+)/SCID(+/+)-Maus abstammt und mit Hepatozyten menschlichen Ursprungs transplantiert ist, die Vermehrungspotential haben, und in der die Hepatozytenpopulation im wesentlichen als die Leber der chimären Maus fungiert;
(ii) Schützen der chimären Maus vor dem Angriff durch ein humanes Komplement, das von den Hepatozyten produziert wird, durch Verabreichen eines Komplementsuppressors oder eines Antikoagulans an die chimäre Maus;
(iii) Analysieren von Metaboliten der chimären Maus und der nichtchimären Maus;
(iv) Analysieren von Metaboliten der Testsubstanz eines Tieres einer anderen Art als der der chimären Maus; und
(v) Vergleichen der Analyseeergebnisse.

## Revendications

1. Procédé pour analyser un métabolite spécifique des humains d'une substance de test, lequel procédé comprend :
(i) l'administration d'une substance de test à une souris chimère et à une souris non chimère de la même espèce ;
la souris chimère étant issue d'une souris uPA-Tg(+/+)/SCID(+/+) et ayant reçu par transplantation des hépatocytes d'origine humaine ayant un potentiel de prolifération, la population d'hépatocytes fonctionnant sensiblement comme le foie de la souris chimère ;
(ii) la protection de la souris chimère contre une attaque par un complément humain produit par les hépatocytes, par administration d'un suppresseur de complément ou d'un anticoagulant à la souris chimère ;
(iii) l'analyse des métabolites provenant de la souris chimère et de la souris non chimère ;
(iv) l'analyse des métabolites de la substance de test provenant d'un animal d'une espèce autre que la souris chimère ; et
(v) la comparaison des résultats analytiques.

2. Procédé pour déterminer un animal présentant un profil métabolique de type humain pour une substance de test, lequel procédé comprend :
(i) l'administration d'une substance de test à une souris chimère et à une souris non chimère de la même espèce ;
la souris chimère étant issue d'une souris uPA-Tg(+/+)/SCID(+/+) et ayant reçu par transplantation des hépatocytes d'origine humaine ayant un potentiel de prolifération, la population d'hépatocytes fonctionnant sensiblement comme le foie de la souris chimère ;
(ii) la protection de la souris chimère contre une attaque par un complément humain produit par les hépatocytes, par administration d'un suppresseur de complément ou d'un anticoagulant à la souris chimère ;
(iii) l'analyse des métabolites provenant de la souris chimère et de la souris non chimère ;
(iv) l'analyse des métabolites de la substance de test provenant d'un animal d'une espèce autre que la souris chimère ; et
(v) la comparaison des résultats analytiques.
